(19) Europäisches Patentamt
European Patent Office
Office européen des brevets

(11) **EP 2 526 926 A1**

(12) **EUROPÄISCHE PATENTANMELDUNG**

(43) Veröffentlichungstag:
28.11.2012 Patentblatt 2012/48

(51) Int Cl.:
*A61K 9/00* (2006.01)    *A61K 31/519* (2006.01)

(21) Anmeldenummer: **11167486.7**

(22) Anmeldetag: **25.05.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Justus-Liebig-Universität Gießen**
**35390 Giessen (DE)**

(72) Erfinder:
• **Schmehl, Thomas**
**35392, Gießen (DE)**

• **Gessler, Tobias**
**35435, Wettenberg (DE)**
• **Beck-Broichsitter, Moritz**
**35039, Marburg (DE)**
• **Kissel, Thomas**
**79219, Staufen (DE)**

(74) Vertreter: **Stumpf, Peter**
**c/o TransMIT GmbH**
**Kerkrader Strasse 3**
**35394 Gießen (DE)**

(54) **Biokompatible Nanopolymerpartikel mit Wirkstoffen für die pulmonale Applikation**

(57)    Die vorliegende Erfindung stellt biokompatible Nanopolymerpartikel bereit, die aus einem biokompatiblen Polymer, einem Stabilisator und einem Wirkstoff zur Behandlung der pulmonalen Hypertonie oder der erektilen Dysfunktion bestehen und zur Herstellung eines Mittels für die Behandlung der pulmonalen Hypertonie oder der erektilen Dysfunktion verwendet werden können.

**Fig. 7**

Printed by Jouve, 75001 PARIS (FR)

## Beschreibung

Die vorliegende Erfindung stellt biokompatible Nanopolymerpartikel mit Wirkstoffen gegen den Lungenhochdruck oder die erektile Dysfunktion bereit, die für die pulmonale Applikation zur Behandlung der pulmonalen Hypertonie oder der erektilen Dysfunktion beim Menschen geeignet sind. Die biokompatiblen Nanopolymerpartikel weisen für die pulmonale Applikation notwendige Vernebelungseigenschaften auf und ermöglichen die gezielte, kontrollierte, verzögerte und langanhaltende Freisetzung der verwendeten Wirkstoffe.

## Beschreibung des allgemeinen Gebietes der Erfindung

Die vorliegende Erfindung betrifft die Gebiete Innere Medizin, Pharmakologie, Nanotechnologie und Medizintechnik.

## Stand der Technik

Die spezifische Wirkstofftherapie des Lungenhochdrucks und der erektilen Dysfunktion umfasst hauptsächlich die intravenöse oder orale Applikation potenter Vasodilatoren. Der Lungenhochdruck ist eine schwerwiegende, lebensbedrohliche Erkrankung, die die körperliche Belastbarkeit stark einschränkt. Der Anstieg des pulmonalen arteriellen Drucks und des vaskulären Widerstands mit nachfolgender Rechtsherz-Dysfunktion resultiert in einer stark reduzierten Lebenserwartung mit einer durchschnittlichen Überlebenszeit von nur 2,8 Jahren nach Diagnosestellung ohne Behandlung.

Zu den Vasodilatoren gehört beispielsweise die Klasse der Phosphodiesterase-Inhibitoren. Phosphodiesterasen sind für den Abbau der intrazellulären Botenstoffe (Second Messenger) zyklisches Adenosinmonophosphat (cAMP) und zyklisches Guanosinmonophosphat (cGMP) verantwortlich. Die Phosphodiesterase-5 ist in der Lage, selektiv cGMP abzubauen. cGMP seinerseits ist der Second Messenger, der durch den endothelialen Relaxationsfaktor Stickstoffmonoxid (NO) aktiviert wird und der an der Relaxation von Blutgefäßen beteiligt ist. Da Phosphodiesterase-5-Inhibitoren die Inaktivierung von cGMP hemmen, führen sie zu einer Verstärkung der gefäßerweiternden Wirkung von Stickstoffmonoxid. Phosphodiesterase-5-Inhibitoren wurden ursprünglich zur Behandlung der Angina pectoris entwickelt, finden aber heute vor allem in der Therapie der erektilen Dysfunktion und der pulmonalen Hypertonie Anwendung. Die Wirkung von Phosphodiesterase-5-Inhibitoren kann insbesondere in Geweben beobachtet werden, welche die Phosphodiesterase-5 stark exprimieren. Dazu zählen neben der glatten Muskulatur der systemischen und pulmonalen Blutgefäße, wo Phosphodiesterase-5-Inhibitoren zu einer Relaxation führen, auch immunkompetente Zellen und Thrombozyten.

Ein Wirkstoff aus der Gruppe der Phosphodiesterase-5-Inhibitoren zur Behandlung des Lungenhochdrucks und der erektilen Dysfunktion ist Sildenafil, das den Patienten als Sildenafil-Citrat (Revatio®) dreimal täglich oral verabreicht wird. Die orale Verabreichung von Sildenafil führt jedoch zu einer systemischen Verfügbarkeit des Wirkstoffs, die mit signifikanten Nebenwirkungen einhergeht. Weitere Phosphodiesterase-Inhibitoren sind Phosphodiesterase-3-Inhibitoren und Phosphodiesterase-4-Inhibitoren.

Phosphodiesterase-3-Inhibitoren (PDE-3-Inhibitoren) sind eine Untergruppe von Medikamenten aus der Gruppe der Phosphodiesterase-Inhibitoren, die zur Therapie der akuten Herzinsuffizienz bei fehlendem Ansprechen von Katecholaminen durch Herabregulieren der Rezeptoren am Myokard zugelassen sind. Bisher zugelassene Arzneistoffe sind Amrinon, Cilostazol, Milrinon und Enoximon. Zur Anwendung am Hund ist der Wirkstoff Pimobendan zugelassen. Durch Hemmung der Phosphodiesterase-3 kommt es zur Erhöhung des Second Messenger cAMP. Außerdem haben die PDE-3-Inhibitoren eine vasodilatatorische Wirkung. Phosphodiesterase-4-Inhibitoren (PDE-4-Inhibitoren) sind Substanzen, die das Enzym Phosphodiesterase-4 hemmen. Die Phosphodiesterase-4 baut die Second Messenger cAMP und cGMP ab. PDE-4-Inhibitoren erhöhen daher die Konzentration von intrazellulärem cGMP (cyclisches Guanosinmonophosphat). Das Enzym kommt u.a. in der Lunge vor. Der Archetyp der Phosphodiesterase-4-Inhibitoren ist Rolipram. PDE-4-Inhibitoren wirken entzündungshemmend und wurden u.a. für die Anwendungsgebiete COPD, Asthma bronchiale, Depression und Multiple Sklerose untersucht. Bislang wurde jedoch nur ein Wirkstoff als Arzneimittel zugelassen: Roflumilast (Daxas®).

Weitere Wirkstoffe zur Behandlung des Lungenhochdrucks sind Aktivatoren und Stimulatoren der löslichen Guanylat-Cyclase. Zur Gruppe der Aktivatoren gehören zum Beispiel Cinaciguat und Ataciguat; zur Gruppe der Stimulatoren gehören zum Beispiel Riociguat, BAY41-2272, BAY41-8543 und CFM-1571.

Darüber hinaus werden auch Endothelin-Rezeptor-Antagonisten zur Behandlung der pulmonalen Hypertonie eingesetzt. Dies sind z.B. Bosentan, Zibotentan, Texosentan, Macitentan, Sitaxentan, Avosentan, Clazosentan, Ambrisentan, Darusentan, Atrasentan, Enrasentan.

Weitere Wirkstoffe zur Behandlung des Lungenhochdrucks sind die Prostanoide. Zu diesen gehören beispielsweise Prostazyklin, Treprostinil und Iloprost.

Als eine selektivere Route der Verabreichung pulmonaler Wirkstoffe ist dem Fachmann die Inhalation bekannt, wodurch unerwünschte systemische Nebenwirkungen umgangen werden können. Die direkte Applikation des Wirkstoffs in die Lunge erleichtert die gezielte Behandlung respiratorischer Erkrankungen, wie es bereits für das Prostazyklin-Analogon Iloprost (Ventavis®) bei der Behandlung der pulmonalen Hypertonie gezeigt worden ist. Die relativ kurze Wirkdauer nach pulmonaler Wirkstoffdeposition ist jedoch ein großer Nachteil der Inhalationstherapie, die eine häufige Wirkstoffverabreichung durch Inhalation erfordert und zu Therapielücken insbesondere während der Nacht führt.

Kolloidale Materialien wie z. B. biokompatible Nanopolymerpartikel sind als geeignete pulmonale Wirkstoffträgersysteme bekannt. Mit dem direkten Transport von in biokompatiblen Nanopolymerpartikeln verkapselten Therapeutika in die Lunge kann eine langanhaltende und kontrollierte Wirkstoff-Freigabe am gewünschten Wirkort erreicht werden, die eine Verlängerung der pharmakologischen Effekte bewirkt.

Die Wahl der Herstellungsmethode der biokompatiblen Nanopolymerpartikel hängt wesentlich von den physikochemischen Parametern des Polymers ab, das benutzt wird, sowie vom Wirkstoff, der in den biokompatiblen Nanopolymerpartikeln verpackt wird. Die Auswahl des Polymers wird von Kriterien wie Biokompatibilität und Biodegradierbarkeit bestimmt. Zudem müssen die biokompatiblen Nanopolymerpartikel weitere Standards erfüllen, wie beispielsweise eine ausreichende Assoziation des therapeutischen Agens mit dem Trägermaterial sowie Stabilität gegenüber den Kräften, die bei der Vernebelung auftreten. Diese stringenten Anforderungen werden von nanopartikulären Wirkstofftransportsystemen, die aus biokompatiblen Polymeren bestehen, erfüllt.

Die Lösungsmittelverdampfungsmethode (Evaporationsmethode) ist als geeignetes Herstellungsverfahren für biokompatible Nanopolymerpartikel bekannt. Diese Methode umfasst das Emulgieren einer organischen Polymerlösung in einer wässrigen Phase mit Stabilisierungs-Hilfsstoff. Es ist dem Fachmann zwar bekannt, dass die verwendeten Stabilisatoren die physikochemischen und biologischen Eigenschaften der verwendeten Formulierungen von biokompatiblen Nanopolymerpartikeln modulieren, jedoch ist die genaue Bedeutung dieser Formulierungsparameter auf die aerodynamischen Charakteristika vernebelter Formulierungen und die Stabilität der biokompatiblen Nanopolymerpartikel bislang nicht bekannt.

Zusammenfassend offenbart der Stand der Technik geeignete Wirkstoffe zur Behandlung der pulmonalen Hypertonie oder erektilen Dysfunktion, deren pharmakologischer Effekt bei pulmonaler Applikation jedoch nur sehr kurz ist und/oder bei systemischer (oral, subkutan, intravenös, etc.) Applikation mit signifikanten Nebenwirkungen einhergeht. Des Weiteren weist der Stand der Technik Nachteile bezüglich aerodynamischer Charakteristika und Stabilität vernebelter Formulierungen von biokompatiblen Nanopolymerpartikeln auf.

## Aufgabe

Aufgabe der Erfindung ist es, ein vernebelbares und inhalierbares Mittel zur Behandlung der pulmonalen Hypertonie oder der erektilen Dysfunktion bereitzustellen, das einen Wirkstoff für die pulmonale Hypertonie oder die erektile Dysfunktion enthält, eine langanhaltende und kontrollierte Freisetzung des Wirkstoffs ermöglicht und für die Anwendung am Menschen geeignet ist.

## Lösung der Aufgabe

Die Aufgabe der Bereitstellung eines Mittels für die Behandlung der pulmonalen Hypertonie oder erektilen Dysfunktion wird erfindungsgemäß gelöst durch biokompatible Nanopolymerpartikel bestehend aus einem biokompatiblen Polymer und einem Stabilisator sowie einem Wirkstoff ausgewählt aus der Gruppe der Phosphodiesterase-Inhibitoren (PDE-Inhibitoren) oder der Guanylat-Cyclase-Aktivatoren oder Guanylat-Cyclase-Stimulatoren oder der Endothelin-Rezeptor-Antagonisten oder der Prostanoide.

Die erfindungsgemäßen biokompatiblen Nanopolymerpartikel können mit der Emulsionsmethode mit anschließender Lösungsmittelverdampfung (Evaporation) hergestellt werden. Die gebildeten dünnen protektiven Stabilisator-Schichten, die zum Beispiel aus Polyvinylalkohol (PVA) bestehen, auf den erfindungsgemäßen biokompatiblen Nanopolymerpartikeln verbessern die Stabilität der Partikel bei der Vernebelung. Die Suspension der erfindungsgemäßen biokompatiblen Nanopolymerpartikel kann in ein Aerosol verwandelt werden, das für die Deposition in der Lunge geeignet ist. Die physiko-chemischen Eigenschaften (z. B. Größe, Oberflächenladung, Wirkstoff-Beladung etc.) der erfindungsgemäßen biokompatiblen Nanopolymerpartikel werden durch die Vernebelung nicht beeinflusst. Die verlängerte Wirkstoff-Freigabe, die mit diesem neuen pulmonalen Wirkstoff-Transportsystem für Wirkstoffe der pulmonalen Hypertonie erreicht wird, führt zu einer Verminderung der Häufigkeit der Wirkstoffgabe gegenüber herkömmlich verwendeten pharmazeutischen Mitteln, was die Lebensqualität und Therapietreue der Patienten verbessert. Zusammenfassend stellen die erfindungsgemäßen biokompatiblen Nanopolymerpartikel ein vielversprechendes Mittel für die Therapie des Lungenhochdrucks oder der erektilen Dysfunktion dar.

Die erfindungsgemäßen biokompatiblen Nanopolymerpartikel bestehen aus einem biokompatiblen Polymer sowie aus einem Stabilisator und einem Wirkstoff zur Behandlung der pulmonalen Hypertonie oder erektilen Dysfunktion ausgewählt aus der Gruppe der Phosphodiesterase-Inhibitoren (PDE-Inhibitoren) oder der Guanylat-Cyclase-Aktivatoren oder Guanylat-Cyclase-Stimulatoren oder der Endothelin-Rezeptor-Antagonisten oder der Prostanoide.

Das biokompatible Polymer ist beispielsweise ein Polyester, Polyanhydrid, Polyorthoester, Polyphosphorester, Polycarbonat, Polyketal, Polyharnstoff, Polyurethan, Blockcopolymer (PEG-PLGA), Sternpolymer oder Kammpolymer.

Der Polyester ist bevorzugt ein lineares Poly(laktid-co-glykolid)-Copolymer (PLGA-Copolymer). Das Kammpolymer ist bevorzugt ein ladungsmodifiziertes verzweigtes Poly(vinylsulfonat-co-vinylalkohol)-graft-poly(D,L-laktid-co-glykolid)-Copolymer (P(VS-VA)-g-PLGA) oder Sulfobutyl-polyvinylalkohol-graft-poly(laktid-co-glykolid)-Copolymer (SB-PVA-g-PLGA).

Es gibt geeignete PLGA-Copolymere für die Herstellung von biokompatiblen Nanopolymerpartikeln, die für die kontrollierte Wirkstoff-Freisetzung ("controlled release") verwendet werden. Diese umfassen beispielsweise, aber nicht erschöpfend, Copolymere aus der Resomer®-Familie. In einer bevorzugten Ausführungsform der Erfindung enthalten die biokompatiblen Nanopolymerpartikel eine der folgenden Resomer®-Substanzen Resomer® Condensate RG 50:50 $M_n$ 2300, Resomer® R202S, Resomer® R202H, Resomer® R203S, Resomer® R203H, Resomer® R207S, Resomer® RG502H, Resomer® RG503H, Resomer® RG504H, Resomer® RG502, Resomer® RG503, Resomer® RG504, Resomer® RG653H, Resomer® RG752H, Resomer® RG752S, Resomer® RG753S, Resomer® RG755S, Resomer® RG756S oder Resomer® RG858S. In einer besonders bevorzugten Ausführungsform enthalten die erfindungsgemäßen biokompatiblen Nanopolymerpartikel das PLGA-Copolymer Resomer® RG502H.

Geeignete P(VS-VA)-g-PLGA-Copolymere für die Herstellung von biokompatiblen Nanopolymerpartikeln sind beispielsweise P(VS-VA)-g-PLGA 2-10, P(VS-VA)-g-PLGA 4-10, P(VS-VA)-g-PLGA 6-5, P(VS-VA)-g-PLGA 6-10, P(VS-VA)-g-PLGA 6-15 oder P(VS-VA)-g-PLGA 8-10.

Des Weiteren beschreibt der Stand der Technik auch geeignete Stabilisatoren, die für die Herstellung von biokompatiblen Nanopolymerpartikeln, welche für die kontrollierte Wirkstoff-Freisetzung ("controlled release") geeignet sind, verwendet werden können. Erfindungsgemäß ist der Stabilisator ausgewählt aus der Gruppe der nichtionischen Tenside, anionischen Tenside, amphoteren Tenside oder der Polymere. Nichtionische Tenside sind beispielsweise, aber nicht erschöpfend, Tween, Span oder Pluronic. Anionisches Tensid ist beispielsweise, aber nicht erschöpfend, Natriumdodecylsulfat (SDS), ein amphoteres Tensid ist beispielsweise, aber nicht erschöpfend, Lecithin. Geeignete Polymere sind beispielsweise die hydrophilen Polymere Polyethylenglykol (PEG), Polyethylenimin (PEI), Polyvinylalkohol (PVA), Polyvinylacetat, Polyvinylbutyrat, Polyvinylpyrrolidon (PVP) oder Polyacrylat sowie natürliche Polymere wie Proteine (z.B. Albumin), Cellulosen und deren Ester und Ether, Amylose, Amylopektin, Chitin, Chitosan, Collagen, Gelatine, Glykogen, Polyaminosäuren (z.B. Polylysin), Stärke, modifizierte Stärken (z.B. HES), Dextrane oder Heparine.

In einer bevorzugten Ausführungsform enthalten die erfindungsgemäßen biokompatiblen Nanopolymerpartikel Polyvinylalkohol, nachfolgend kurz als PVA bezeichnet, als Stabilisator. PVA ist ein kristalliner, wasserlöslicher Kunststoff.

Die erfindungsgemäßen biokompatiblen Nanopolymerpartikel enthalten des Weiteren einen Wirkstoff für die pulmonale Hypertonie oder erektile Dysfunktion ausgewählt aus der Gruppe der Phosphodiesterase-Inhibitoren (PDE-Inhibitoren) oder der Guanylat-Cyclase-Aktivatoren oder Guanylat-Cyclase-Stimulatoren oder der Endothelin-Rezeptor-Antagonisten oder der Prostanoide. Zu den PDE-Inhibitoren, welche für die Behandlung der pulmonalen Hypertonie und der erektilen Dysfunktion geeignet sind, zählen unter anderem die Phosphodiesterase-5-Inhibitoren (PDE-5-Inhibitoren). PDE-5-Inhibitoren sind Substanzen, die das cGMP-abbauende Enzym Phosphodiesterase 5 (PDE-5) hemmen und somit die Konzentration von intrazellulärem cGMP (zyklisches Guanosinmonophosphat) erhöhen. Sie verursachen unter anderem eine Erweiterung von Blutgefäßen (Vasodilatation). Auf Grund der Selektivität für die Isoform 5 der Phosphodiesterase kann unterschieden werden zwischen nichtselektiven Phosphodiesterase-Inhibitoren, wie die Methylxanthine Koffein, Theophyllin, Theobromin, die unspezifisch verschiedene Phosphodiesterasen hemmen, und den selektiven Inhibitoren der Phosphodiesterase 5, wie beispielsweise den Wirkstoffen Sildenafil, Tadalafil und Vardenafil. In einer bevorzugten Ausführungsform enthalten die erfindungsgemäßen biokompatiblen Nanopolymerpartikel den Wirkstoff Sildenafil. Sildenafil ist dem Fachmann auch unter der chemischen Formel 5-[2-ethoxy-5-(4-methyl-1-piperazinylsulphonyl)phenyl]-1-methyl-3-n-propyl-1,6-dihydro-7H-pyrazolo[4,3-d]pyrimidin-7-one bekannt. In einer besonders bevorzugten Ausführungsform handelt es sich bei dem Sildenafil um die freie Base.

Zu den PDE-Inhibitoren gehören neben den PDE-5-Inhibitoren des Weiteren die Phosphodiesterase-3-Inhibitoren (PDE-3-Inhibitoren) und die Phosphodiesterase-4-Inhibitoren (PDE-4-Inhibitoren). PDE-3-Inhibitoren werden zur Therapie der akuten Herzinsuffizienz bei fehlendem Ansprechen von Katecholaminen eingesetzt. Durch Hemmung der Phosphodiesterase-3 kommt es zur Erhöhung des second Messenger cAMP. Außerdem haben die PDE-3-Inhibitoren eine vasodilatatorische Wirkung. PDE-4-Inhibitoren sind Substanzen, die das Enzym Phosphodiesterase-4 hemmen. Die Phosphodiesterase-4 baut die second Messenger cAMP und cGMP ab. PDE-4-Inhibitoren erhöhen daher die Konzentration von intrazellulärem cGMP (cyclisches Guanosinmonophosphat). Das Enzym kommt u.a. in der Lunge vor. PDE-4-Inhibitoren wirken entzündungshemmend.

Weitere geeignete Wirkstoffe für die Behandlung der pulmonalen Hypertonie sind Guanylat-Cyclase-Aktivatoren oder Guanylat-Cyclase-Stimulatoren und Endothelin-Rezeptor-Antagonisten.

Guanylat-Cyclase-Aktivatoren sind zum Beispiel Cinaciguat und Ataciguat; Guanylat-Cyclase-Stimulatoren

**4**

sind Riociguat, BAY41-2272, BAY41-8543 und CFM-1571. Endothelin-Rezeptor-Antagonisten sind beispielweise Bosentan, Zibotentan, Texosentan, Macitentan, Sitaxentan, Avosentan, Clazosentan, Ambrisentan, Darusentan, Atrasentan, Enrasentan.

Weitere geeignete Wirkstoffe zur Behandlung der pulmonalen Hypertonie sind die Prostanoide, zu denen beispielsweise Prostazyklin, Treprostinil und Iloprost gehören.

Charakterisierung der erfindungsgemäßen biokompatiblen Nanopolymerpartikel

Die erfindungsgemäßen biokompatiblen Nanopolymerpartikel haben einen mittleren geometrischen Durchmesser zwischen 10 nm bis 10 $\mu$m, so dass sie gut vernebelbar sind, und eine Stabilisator-Schichtdicke zwischen 0 bis 50 nm. Die Stabilisator-Schichtdicke ist dabei jedoch nicht größer als der mittlere geometrische Radius der biokompatiblen Nanopolymerpartikel. In einer bevorzugten Ausführungsform haben die biokompatiblen Nanopolymerpartikel einen mittleren geometrischen Durchmesser von 500 nm bis 5 $\mu$m, um eine länger dauernde Wirkstoff-Freisetzung zu ermöglichen, oder zwischen 50 nm und 250 nm, um eine Aufnahme der Partikel in Makrophagen zu verhindern.

Des Weiteren weisen die erfindungsgemäßen biokompatiblen Nanopolymerpartikel vorzugsweise eine negative Oberflächenladung und ein negatives Zeta-Potenzial auf. Die biokompatiblen Nanopolymerpartikel können aber alternativ auch eine positive Oberflächenladung und ein positives Zeta-Potenzial besitzen.

Erfindungsgemäß enthalten die biokompatiblen Nanopolymerpartikel zwischen 0 bis 50 % (w/w) und in einer bevorzugten Ausführungsform zwischen 1 bis 20 % (w/w) eines Wirkstoffs zur Behandlung der pulmonalen Hypertonie oder der erektilen Dysfunktion.

Die erfindungsgemäßen biokompatiblen Nanopolymerpartikel sind vorteilhaft mit piezoelektrischen, Düsen-, Ultraschall-Aerosolgeneratoren, Soft-Mist-Inhalern, Metered Dose Inhalern oder Dry Powder Inhalern vernebelbar, d.h. die Applikation in die Lunge erfolgt durch Inhalation eines Aerosols (Suspension, Pulver) mit Hilfe eines Aerosolgenerators. Eine weitere Möglichkeit der Applikation in die Lunge besteht in der Instillation, beispielsweise über einen Katheter, ein Bronchoskop oder einen Beatmungszugang (z.B. Tubus oder Trachealkanüle).

Herstellung der erfindungsgemäßen biokompatiblen Nanopolymerpartikel

Die erfindungsgemäßen biokompatiblen Nanopolymerpartikel werden beispielsweise mit Hilfe der Emulsionsmethode und anschließender Lösungsmittelverdampfung (Evaporationsmethode) hergestellt. Die erfindungsgemäßen biokompatiblen Nanopolymerpartikel bestehen aus einem biokompatiblen Polymer sowie aus einem Stabilisator und einem Wirkstoff zur Behandlung der pulmonalen Hypertonie oder der erektilen Dysfunktion. Das biokompatible Polymer der erfindungsgemäßen Nanopolymerpartikel ist beispielsweise ein Polyester (PLGA, PLA), Polyanhydrid, Polyorthoester, Polyphosphorester, Polycarbonat, Polyketal, Polyharnstoff, Polyurethan, Blockcopolymer (PEG-PLGA), Sternpolymer oder Kammpolymer. Erfindungsgemäß ist der Stabilisator ausgewählt aus der Gruppe der nichtionischen Tenside, anionischen Tenside, amphoteren Tenside oder der Polymere. Der Wirkstoff der erfindungsgemäßen biokompatiblen Nanopolymerpartikel ist ausgewählt aus der Gruppe der Phosphodiesterase-Inhibitoren (PDE-Inhibitoren) oder Guanylat-Cyclase-Aktivatoren oder Guanylat-Cyclase-Stimulatoren oder Endothelin-Rezeptor-Antagonisten oder der Prostanoide.

Alternativ werden biokompatible Nanopolymerpartikel auch mittels Nanopräzipitation, Aussalzen, Polymerisation oder Sprühtrocknung hergestellt. Diese genannten Herstellungsmethoden sind dem Fachmann bekannt.

Werden die Partikel mittels der Evaporationsmethode hergestellt, so wird zunächst das Polymer unter Zugabe eines Wirkstoffs zur Behandlung der pulmonalen Hypertonie oder der erektilen Dysfunktion in einem Lösungsmittel gelöst. Dabei beträgt die Konzentration des eingesetzten Wirkstoffs zwischen 7% und 20% bezogen auf das Polymer, um einen theoretischen Wirkstoff-Gehalt der Partikel von 5% zu erhalten. Anschließend wird die organische Phase in ein konstantes Volumen wässriger Phase, die einen Stabilisator enthält, überführt. Nach Vermischen der beiden Phasen und Beschallung mit Ultraschall wird anschließend das organische Lösungsmittel durch Verdampfung entfernt und die Partikel in Suspension erhalten. Geeignete Lösungsmittel, in welchen das erfindungsgemäß verwendete Polymer zu mindestens 0,1 % (w/w) löslich ist, sind beispielsweise, aber nicht erschöpfend, Dichlormethan, Chloroform, Ethylacetat, Benzylalkohol, Methylethylketon, Propylencarbonat. In einer bevorzugten Ausführungsform wird Polyvinylalkohol (PVA) als Stabilisator verwendet.

In einer bevorzugten Ausführungsform werden für die Herstellung der erfindungsgemäßen biokompatiblen Nanopolymerpartikel biokompatibles Polymer zwischen 1 bis 100 g/l und Stabilisator zwischen 0,1 bis 25 g/l verwendet. In einer besonders bevorzugten Ausführungsform beträgt für die Herstellung die biokompatible Polymer-Konzentration 50 g/l und die Stabilisator-Konzentration 10 g/l.

Handelt es sich bei dem verwendeten Wirkstoff um den PDE-5-Inhibitor Sildenafil, so erfolgt die Herstellung der biokompatiblen Nanopolymerpartikel in Gegenwart von Sildenafil in einer wässrigen Phase bei einem pH-Wert zwischen 2 bis 10. Sildenafil ist ein amphoterischer Wirkstoff mit einem pH-abhängigen Löslichkeitsprofil und limitierter Löslichkeit bei neutralen pH-Werten.

Eine alternative Herstellungsmethode für die erfindungsgemäßen biokompatiblen Nanopolymerpartikel ist die Sprühtrocknung. Dazu werden zwischen 0,1% bis 10% des Polymers mit oder ohne Zugabe zwischen 1% bis 20% (bezogen auf das verwendete Polymer) eines Wirkstoffs für die pulmonale Hypertonie oder der erektilen Dysfunktion, wie beispielsweise dem PDE-5-Inhibitor Sildenafil, in einem nicht mit Wasser mischbaren Lösungsmittel, wie zum Beispiel Methylenchlorid, gelöst. Anschließend wird diese Lösung nach Filtration mit einem Sprühtrockner, wie zum Beispiel einem Nano-Sprühtrockner, nach Angaben des Herstellers sprühgetrocknet. Alternativ kann sich die Sprühtrocknung unter Verwendung des Sprühtrockners an die Herstellungsschritte der Emulsionsmethode mit anschließender Lösungsmittelverdampfung, der Nanopräzipitation, des Aussalzens oder der Polymerisation anschließen.

Verwendung der erfindungsgemäßen biokompatiblen Nanopolymerpartikel

Die erfindungsgemäßen biokompatiblen Nanopolymerpartikel können zur Herstellung eines pharmazeutischen Mittels zur Behandlung der pulmonalen Hypertonie oder der erektilen Dysfunktion eingesetzt werden. Biokompatibilität meint dabei die Verträglichkeit für das Gewebe und die Zellen am Wirkort, z.B. der Lunge.

Die Wirkung der erfindungsgemäßen biokompatiblen Nanopolymerpartikel beruht auf dem darin enthaltenen Wirkstoff zur Behandlung der pulmonalen Hypertonie oder der erektilen Dysfunktion, der kontrolliert, kontinuierlich und langanhaltend über einen Zeitraum bis zu 48 Stunden aus den biokompatiblen Nanopolymerpartikeln an der Lunge oder den Bronchien oder den Grenzflächen der Lunge freigesetzt wird.

Sämtliche aus den Ansprüchen, der Beschreibung und den Zeichnungen hervorgehenden Merkmale und Vorteile einschließlich konstruktiver Einzelheiten, räumlicher Anordnungen und Verfahrensschritte können sowohl für sich als auch in den verschiedensten Kombinationen erfindungswesentlich sein.

**Ausführungsbeispiele**

Die folgenden Ausführungsbeispiele 1 und 2 beschreiben die Herstellung bzw. die Charakterisierung von erfindungsgemäßen biokompatiblen Nanopolymerpartikeln. In den Ausführungsbeispielen wird der PDE-5-Inhibitor Sildenafil als Wirkstoff verwendet und ist dementsprechend als Beispiel für einen PDE-5-Inhibitor zu betrachten. Die erfindungsgemäßen biokompatiblen Nanopolymerpartikel werden im Folgenden in Kurzform auch als Partikel bezeichnet. Poly(D,L-laktid-*co*-glykolid)-Copolymer (PLGA) oder Poly(vinylsulfonat-co-vinylalkohol)-graft-poly(D,L-laktid-co-glykolid)-Copolymer (P(VS-VA)-g-PLGA) werden nachfolgend auch kurz als Polymer bezeichnet.

1. Ausführungsbeispiel 1

*1.1 Herstellung der erfindungsgemäßen biokompatiblen Nanopolymerpartikel gemäß Anspruch 14 mittels Emulsion und anschließender Evaporation*

Die erfindungsgemäßen biokompatiblen Nanopolymerpartikel werden beispielsweise bei Raumtemperatur mittels der Emulsionsmethode mit anschließender Lösungsmittelverdampfung, welche dem Fachmann bekannt ist, synthetisiert. Zuerst wird dazu zwischen 1 bis 100 g/l Poly(D,L-laktid-*co*-glykolid)-Copolymer (PLGA), das kommerziell erhältlich ist und beispielsweise als Resomer® RG502H, RG502, RG503H oder RG504H von Boehringer Ingelheim (Ingelheim, Deutschland) bezogen werden kann, oder Poly(vinylsulfonat-co-vinylalkohol)-*graft*-poly(D,L-laktid-*co*-glykolid)-Copolymer (P(VS-VA)-g-PLGA) mit oder ohne Zugabe von zwischen 1% bis 20% eines Wirkstoffs zur Behandlung der pulmonalen Hypertonie oder der erektilen Dysfunktion, wie beispielsweise der PDE-5-Inhibitor Sildenafil, welcher als freie Base kommerziell zum Beispiel von AK Scientific (Mountain View, Kalifornien, USA) erhältlich ist, in einem nicht mit Wasser mischbaren Lösungsmittel, wie zum Beispiel Methylenchlorid, gelöst. Um einen theoretischen Gehalt von 5% Wirkstoff der erfindungsgemäßen biokompatiblen Nanopartikel zu erhalten, werden zwischen 7% bis 20% des Wirkstoffs bezogen auf das verwendete Polymer verwendet. Dann werden 2 ml der organischen Phase (dispergierte Phase) in 10 ml einer beispielsweise mit 1 mM 4-(2-Hydroxyethyl)piperazin-1-ethansulfonsäure auf pH 8 eingestellten wässrigen Phase (konstante Phase) enthaltend zwischen 0,1 bis 15 g/l eines Oberflächen-Stabilisators, der beispielsweise Polyvinylalkohol (PVA) ist, überführt. PVA ist kommerziell beispielsweise als Mowiol 4-88® von Sigma-Aldrich (Steinheim, Deutschland) erhältlich. Nach Vermischen der beiden Phasen wird die Emulsion mit Ultraschall behandelt. Die organische Phase wird anschließend langsam mit einem Rotationsevaporator durch Lösungsmittelverdampfung entfernt. Die Partikel werden direkt nach der Herstellung verwendet. Alternativ schließt sich an die Herstellung eine Sprühtrocknung der erfindungsgemäßen biokompatiblen Nanopolymerpartikel an. Dazu werden die erfindungsgemäßen biokompatiblen Nanopolymerpartikel (0,2% bis 2%) nach Filtration mit einem Sprühtrockner, wie zum Beispiel dem Nano-Sprühtrockner B-90 (Büchi, Flawil, Schweiz) nach Angaben des Herstellers sprühgetrocknet.

*1.2 Herstellung der erfindungsgemäßen biokompatiblen Nanopolymerpartikel gemäß Anspruch 15 mittels Sprühtrocknung*

Die erfindungsgemäßen biokompatiblen Nanopolymerpartikel werden beispielsweise mittels Sprühtrocknung hergestellt. Dazu werden zwischen 0,1% bis 10% Poly(D,L-laktid-*co*-glykolid)-Copolymer (PLGA), das kommerziell erhältlich ist und beispielsweise als Resomer® RG502H von Boehringer Ingelheim (Ingelheim, Deutschland) bezogen werden kann, mit oder ohne Zugabe von zwischen 1% bis 20% (bezogen auf das verwendete Polymer) eines Wirkstoffs zur Behandlung der pulmonalen Hypertonie oder der erektilen Dysfunktion, wie beispielsweise der PDE-5-Inhibitor Sildenafil, welcher als freie Base kommerziell zum Beispiel von AK Scientific (Mountain View, Kalifornien, USA) erhältlich ist, in einem nicht mit Wasser mischbaren Lösungsmittel, wie zum Beispiel Methylenchlorid, gelöst. Anschließend wird diese Lösung nach Filtration mit einem Sprühtrockner, wie zum Beispiel dem Nano-Sprühtrockner B-90 (Büchi, Flawil, Schweiz), nach Angaben des Herstellers sprühgetrocknet.

2. Ausführungsbeispiel 2

*Charakterisierung der erfindungsgemäßen biokompatiblen Nanopolymerpartikel*

Die gemäß Ausführungsbeispiel 1.1 oder 1.2 hergestellten biokompatiblen Nanopolymerpartikel werden mit Hilfe der nachfolgend unter Ausführungsbeispiel 2, Punkte 2.1 bis 2.4, beschriebenen Methoden und Ergebnisse charakterisiert. Dazu werden diese entweder direkt nach der Herstellung oder nach Vernebelung mit einem Vernebler, beispielsweise dem Aeroneb® Professional von Aerogen (Dangan, Galway, Irland), nach Herstellerangaben verwendet.

*2.1 Durchmesser, Größenverteilung und Oberflächenladung der erfindungsgemäßen biokompatiblen Nanopolymerpartikel*

Frisch hergestellte biokompatible Nanopolymerpartikel, die mit Hilfe der Emulsionsmethode mit anschließender Lösungsmittelverdampfung wie unter Ausführungsbeispiel 1.1 beschrieben hergestellt werden, werden in verschiedenen Kombinationen der Polymer-Konzentration (zwischen 5 bis 100 g/l) und der PVA-Konzentration (zwischen 1 bis 50 g/l) auf ihre Eigenschaften Durchmesser, Größenverteilung und Oberflächenladung untersucht. Der hydrodynamische Durchmesser und die Größenverteilung (Polydispersität, PDI) der biokompatiblen Nanopolymerpartikel werden durch dynamische Lichtstreuung (DLS) gemessen. Das Zeta-Potenzial als Maß für die Oberflächenladung wird durch Laser-Doppler-Anemometrie (LDA), beispielsweise mit einem Zetasizer NanoZS/ZEN3600 (Malvern Instruments, Herrenberg, Deutschland), bestimmt. Alle Messungen werden bei einer Temperatur von 25°C mit Aliquots durchgeführt, die mit filtriertem und doppeltdestilliertem Wasser für die DLS bzw. 1,56 mM NaCl für die LDA verdünnt werden. Alle Messungen werden mindestens in Dreifachbestimmung mit mindestens 10 Durchläufen direkt nach der Präparation der biokompatiblen Nanopolymerpartikel durchgeführt. Dabei gibt n im Folgenden die Anzahl der Bestimmungen an.

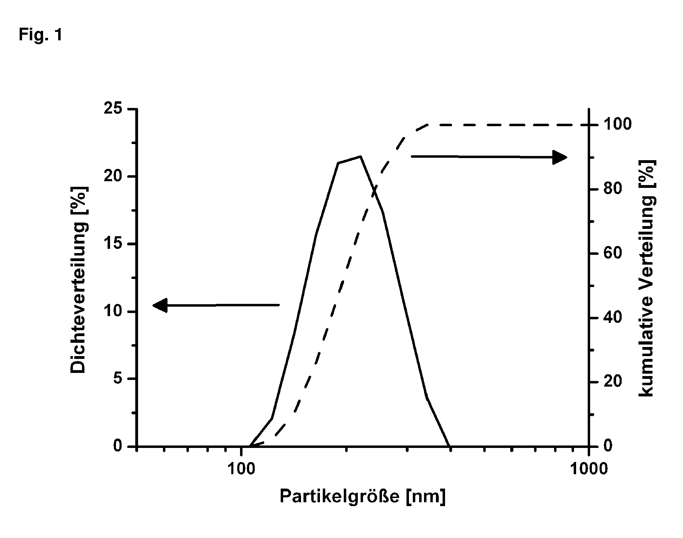

Eine enge Partikelgrößenverteilung, d.h. Polydispersitätsindices (PDI), mit einem Wert kleiner 0,1 werden bei einer PVA-Konzentration größer 5g/l bei gleichbleibender PLGA-Konzentration von 50 g/l oder bei einer PLGA-Konzentration zwischen 10 bis 50 g/l bei gleichbleibender PVA-Konzentration von 10 g/l gefunden. Die Größenverteilung, die mit Hilfe von DLS bestimmt wird, frisch hergestellter biokompatibler Nanopolymerpartikel ist in Fig. 1 dargestellt. Die Größe der biokompatiblen Nanopolymerpartikel beträgt zwischen 100 und 400 nm (schwarze Linie in Abb. 1). Biokompatible Nanopolymerpartikel, die mit einer PLGA-Konzentration von 50g/l und einer PVA-Konzentration von 10 g/l hergestellt werden, weisen eine mittlere Partikelgröße von 195,1 $\pm$ 9,6 nm (Mittelwert $\pm$ Standardabweichung, n=4), eine enge Größenverteilung, d.h. einen kleinen Polydispersitätsindex (PDI), von 0,078 $\pm$ 0,002 (Mittelwert $\pm$ Standardabweichung, n=4) sowie eine negative Oberflächenladung, d.h. ein negatives Zeta-Potenzial, von - 5,7 $\pm$ 0,8 mV (Mittelwert $\pm$ Standardabweichung, n=4) auf.

Um den Durchmesser, die Größenverteilung und die Oberflächenladung als Maß für die Stabilität der biokompatiblen Nanopolymerpartikel nach der Vernebelung zu untersuchen, werden die erfindungsgemäßen biokompatiblen Nanopolymerpartikel mit einem theoretischen Gehalt von 5% (w/w) des Wirkstoffs Sildenafil (freie Base) gemäß Ausführungsbeispiel 1.1 hergestellt und vor und nach Vernebelung mit dem Vernebler Aeroneb® Professional charakterisiert. Dazu werden die vernebelten Suspensionen der biokompatiblen Nanopolymerpartikel gesammelt und qualitativ wie bei Dailey et al. beschrieben (Dailey LA, Kleemann E, Wittmar M et al.: Surfactantfree, biodegradable nanoparticles for aerosol therapy based on the branched polyesters, DEAPA-PVAL-g-PLGA. Pharm. Res. 20(12), 2011-2020 (2003); Dailey LA, Schmehl T, Gessler T et al.: Nebulization of biodegradable nanoparticles: impact of nebulizer technology and nanoparticle characteristics on aerosol features. J. Controlled Release. 86(1), 131-144 (2003)) untersucht. Die Suspensionen der biokompatiblen Nanopolymerpartikel werden bei einer Luftflußrate von 5 l/min vernebelt und durch Anbringen eines Glasobjektträgers direkt vor dem T-förmigen Mundstück des Verneblers, was das Abscheiden der Aerosoltröpfchen auf dem Objektträger ermöglicht, gesammelt. Die daraus resultierende Kondensationsflüssigkeit wird zur weiteren Analyse gesammelt. Die Stabilität der vernebelten biokompatiblen Nanopolymerpartikel wird mit Hilfe von DLS und LDA wie oben beschrieben untersucht.

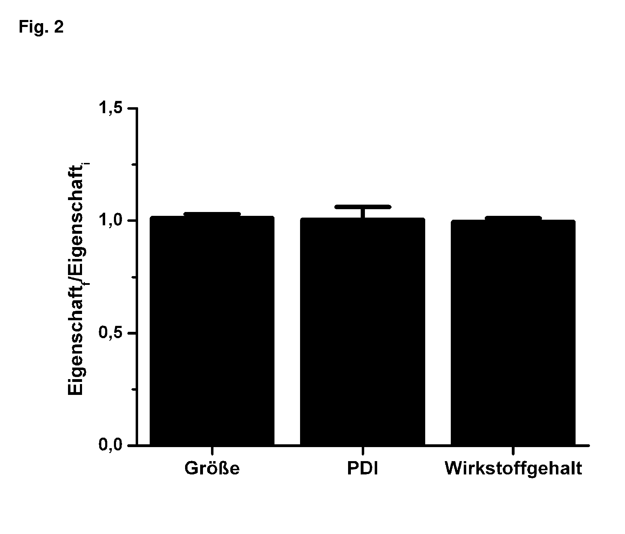

Die erfindungsgemäßen biokompatiblen Nanopolymerpartikel weisen eine durchschnittliche Größe von 197,1 $\pm$ 1,7 nm, eine enge Größenverteilung mit einem PDI von 0,074 $\pm$ 0,005 sowie eine negative Oberflächenladung mit einem Zeta-Potenzial von -5,1 $\pm$ 0,3 mM auf. Die Parameter Partikelgröße, PDI und Sildenafil-Gehalt (siehe 2.3) werden in Fig. 2 als Quotient aus den Werten vor und nach Vernebelung dargestellt, Die Figur zeigt, dass die Vernebelung keinen signifikanten Einfluss auf die genannten Parameter hat.

*2.2 Schichtdicke des Stabilisators der erfindungsgemäßen biokompatiblen Nanopolymerpartikel*

Die Dicke der adsorbierten PVA-Schichten, die als Oberflächenstabilisator der erfindungsgemäßen biokompatiblen Nanopolymerpartikel fungieren, wird mit Hilfe der DLS- und der Zeta-Potenzial- Messungen wie unter Punkt 2.1 beschrieben als Funktion der Elektrolyt-Konzentration bestimmt. Geeignete Bestimmungsmethoden sind dem Fachmann bekannt. In Bezug auf die DLS-Messungen ist die adsorbierte PVA-Schichtdicke ($\delta$) abgeleitet vom Vergleich der Partikelgrößen von blanken ($d_0$) und beschichteten ($d_{ads}$) biokompatiblen Nanopolymerpartikeln gemäß der folgenden Formel (1)

$$\delta = \frac{d_{ads} - d_0}{2} \qquad (1)$$

Die Schichtdicke aus den Zeta-Potenzial Messungen wird mit Hilfe der dem Fachmann bekannten Gouy-Chapman-Näherung kalkuliert, die die Abnahme des elektrostatischen Potenzials als eine Funktion der Distanz von der Oberfläche in der folgenden Formel (2) beschreibt.

$$\psi_x = \psi_0 \cdot e^{-\kappa x} \qquad (2)$$

Dabei ist $\psi_x$ das Potenzial bei einer Distanz x von der Oberfläche, $\psi_0$ ist das Oberflächenpotenzial und $K^{-1}$ ist die Debye-Länge. Eine Erhöhung der Elektrolyt-Konzentration (NaCl) erniedrigt die Debye-Länge. Zeta-Potenziale werden definiert als elektrostatische Potenziale an der Abscherebene, von denen angenommen wird, dass sie nur außerhalb der fixierten wässrigen Schicht eines biokompatiblen Nanopolymerpartikels auftreten. Aus Gleichung (2) folgt Gleichung (3)

$$ln\,\psi_x = ln\,\psi_0 - \kappa \cdot x \qquad (3)$$

Wenn Zeta-Potenziale ($\psi_x$) in verschiedenen Konzentrationen von NaCl (0, 0.1, 0.2, 0.5, 1, 2 und 5 mM) gemessen werden und gegen *K* entsprechend $3.33 \cdot c^{1/2}$ aufgetragen werden, wobei *c* die Molalität von Elektrolyten ist, gleicht der Konzentrations-Anstieg die Dicke der adsorbierten Polymerschichten aus.

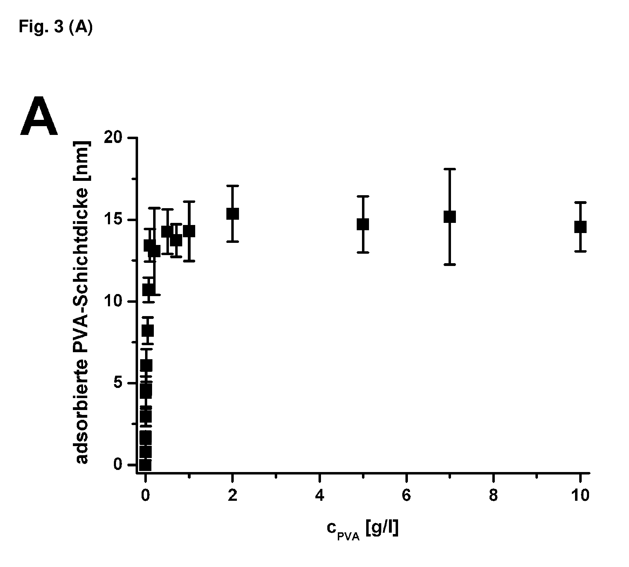

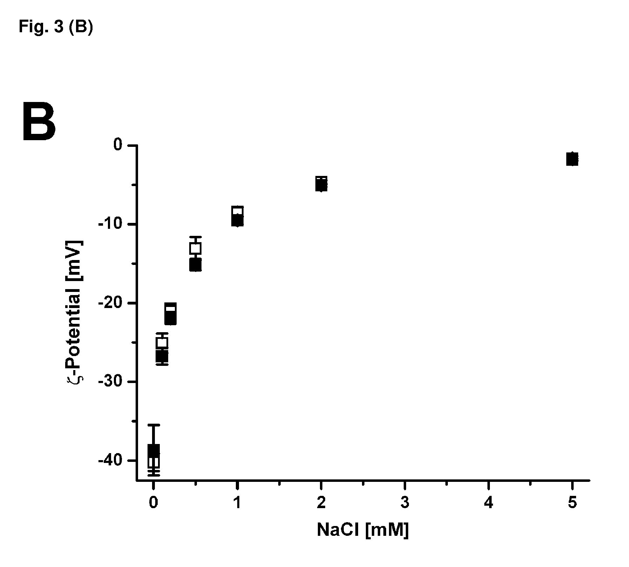

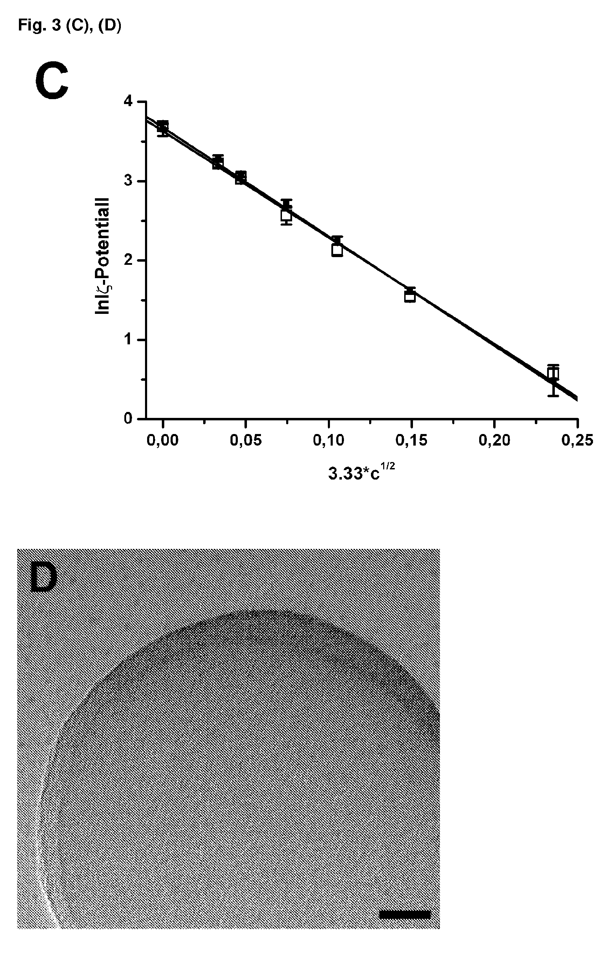

Fig. 3 stellt die adsorbierte PVA-Schichtdicke auf den biokompatiblen Nanopolymerpartikeln für frisch hergestellte (weiße Quadrate) als auch vernebelte Partikel (schwarze Quadrate) dar. In Fig. 3A ist diese in Abhängigkeit von der eingesetzten PVA-Konzentration gezeigt. Die Schichtdicke beträgt sowohl für frisch hergestellte als auch vernebelte Partikel zwischen 10 und 20 nm. Dieses Ergebnis wird auch durch transmissionselektronenmikroskopische Aufnahmen bestätigt. Dazu wird ein Kupfer-Grid (zum Beispiel S160-3, Plano, Wetzlar, Deutschland) mit einer dünnen Schicht einer verdünnten Suspension der biokompatiblen Nanopolymerpartikel beschichtet. Die biokompatiblen Nanopolymerpartikel werden dann auf dem Grid getrocknet und unter einem Transmissionselektronenmikroskop (TEM, beispielsweise JEM-3020 TEM, JEOL, Eching, Deutschland) bei einer Beschleunigungsspannung von 300 kV untersucht. Fig. 3D zeigt eine repräsentative TEM-Aufnahme eines erfindungsgemäßen biokompatiblen Nanopolymerpartikels, bei der die PVA-Schicht (eingesetzte Konzentration bei der Herstellung gemäß Ausführungsbeispiel 1 gleich 10 g/l) deutlich zu erkennen ist. Das Zeta-Potenzial, also die Oberflächenladung, der Partikel ist bei allen getesteten NaCl-Konzentrationen negativ (Fig. 3B). Die gerade Linie in Fig. 3C zeigt den linearen Verlauf der experimentellen Daten.

*2.3 Gehalt des Wirkstoffs in den erfindungsgemäßen biokompatiblen Nanopolymerpartikeln*

Um den Gehalt an PDE-5-Inhibitor der gemäß Ausführungsbeispiel 1 hergestellten biokompatiblen Nanopolymerpartikel zu bestimmen, wird beispielsweise 1 ml der Suspension der biokompatiblen Nanopolymerpartikel bei 16873 x g für 30 min bei 25°C zentrifugiert. Anschließend wird der Überstand vorsichtig abgenommen und darin die Menge von nicht eingeschlossenem PDE-5-Inhibitor bestimmt. Die Pellets aus der Zentrifugation werden gefriergetrocknet, gewogen und anschließend beispielsweise in Chloroform, das als Lösungsmittel für PLGA und Sildenafil geeignet ist, gelöst. Die nicht gelöste Fraktion (Stabilisator) wird durch Zentrifugation abgetrennt. Anschließend wird aus der organischen Phase ein Aliquot entnommen, um die Menge an eingeschlossenem PDE-5-Inhibitor zu bestimmen. Die Konzentration des PDE-5-Inhibitors wird durch UV/Vis-Spektroskopie mit einem Spektrophotometer (beispielsweise Ultrospec® 3000, Pharmacia Biotech, Freiburg, Deutschland) bestimmt. Die Absorption aller Aliquots wird bei einer Wellenlänge von 291 nm gemessen. Der Gehalt an PDE-5-Inhibitor (PDE5H) in den biokompatiblen Nanopolymerpartikeln (PLGA-BNPP) wird anhand einer Kalibrierungskurve kalkuliert und ist in der folgenden Formel (4) definiert.

$$PDE5H Gehalt\ (\%\ (w/w)) = \frac{Masse\ PDE5H\ in\ PLGA - BNPP}{Masse\ PLGA - BNPP} \cdot 100 \qquad (4)$$

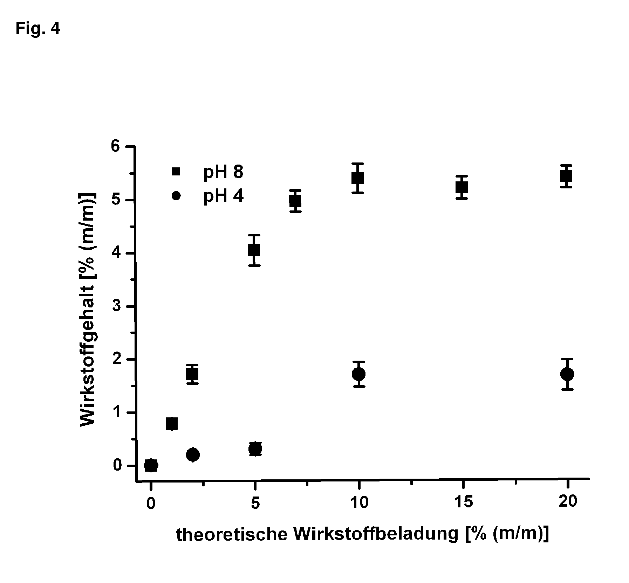

Die erfindungsgemäßen biokompatiblen Nanopolymerpartikel werden mit einem theoretischen Gehalt von 5% (w/w) des Wirkstoffs Sildenafil (freie Base) gemäß Ausführungsbeispiel 1 mit 1% PVA hergestellt und charakterisiert. Der tatsächliche Gehalt der erfindungsgemäßen biokompatiblen Nanopolymerpartikel an Sildenafil liegt bei 4,05 ± 0,15% (w/w) und ist als Funktion des theoretischen Wirkstoff-Gehalts in Abhängigkeit vom pH-Wert in Fig. 4 gezeigt. Der Wirkstoff-Gehalt der Partikel ist demnach bei pH 4 (schwarze Kreise) mit maximal 2% (w/w) deutlich geringer als bei pH 8 mit maximal 5,5% (w/w) (schwarze Quadrate).

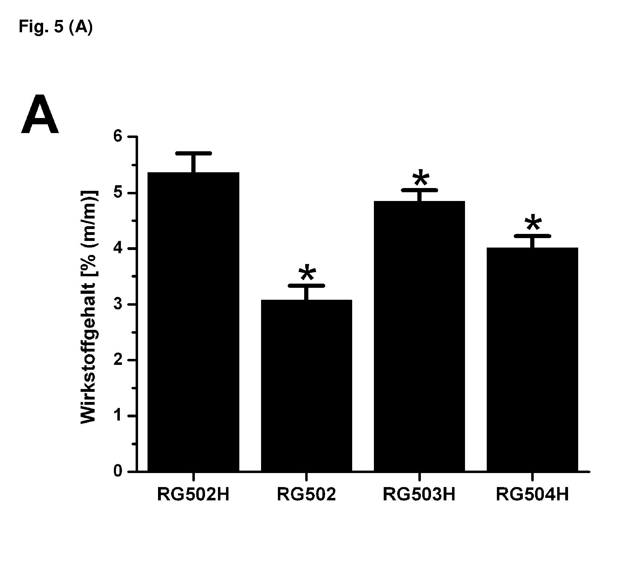

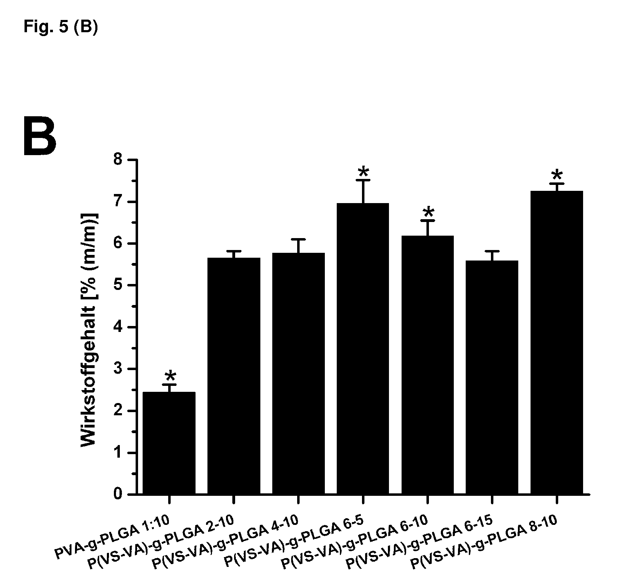

Der Wirkstoffgehalt in Abhängigkeit vom ausgewählten linearen PLGA-Copolymer oder verzweigten P(VA-VS)-g-PLGA-Copolymer bei einem theoretischen Wirkstoffgehalt von 10% ist in Fig. 5 gezeigt. Bei den linearen PLGA-Copolymeren weisen diejenigen erfindungsgemäßen biokompatiblen Nanopolymerpartikel den größten Gehalt mit 5 bis 5,5% (w/w) Sildenafil auf, die gemäß Ausführungsbeispiel 1 mit dem PLGA-Copolymer Resomer® RG502H hergestellt werden (A). Bei den verzweigten P(VS-VA)-g-PLGA-Copolymeren liegt der Gehalt an Sildenafil in Abhängigkeit von der Viskosität der organischen Polymerlösung und der Polymerladung zwischen 5% bis 8% (w/w) (B).

Der Sildenafil-Gehalt ist zusammen mit den Parametern Partikelgröße und PDI (siehe 2.1) in Fig. 2 als Quotient aus den Werten vor und nach Vernebelung dargestellt, Die Figur zeigt, dass die Vernebelung keinen signifikanten Einfluss auf den Sildenafil-Gehalt hat.

*2.4 Freisetzung des Wirkstoffs aus den erfindungsgemäßen biokompatiblen Nanopolymerpartikeln*

Untersuchungen zur Freisetzung des Wirkstoffs PDE-5-Inhibitor *in vitro* werden in phosphatgepufferter Salzlösung bei einem pH-Wert von beispielsweise 7,4 500 Minuten lang bei 37°C durchgeführt. Die Studien werden mit biokompatiblen Nanopolymerpartikeln durchgeführt, die einen theoretischen PDE-5-Inhibitor-Gehalt von 5% (w/w) aufweisen. Aliquots der Suspensionen der biokompatiblen Nanopolymerpartikel werden in Glasröhrchen überführt und mit Medium, bestehend aus phosphatgepufferter Salzlösung (PBS) pH 7,4 + 0,1% Natriumdodecylsulfat (SDS), verdünnt. Die nachfolgende Inkubation erfolgt bei 37°C unter Schütteln der Aliquots. Parallel zu dem Versuchsansatz wird der PDE-5-Inhibitor allein in Medium unter den gleichen Bedingungen inkubiert. Eine Teilmenge wird zu vorgegebenen Zeitpunkten entnommen und zentrifugiert. Die Freisetzung von PDE-5-Inhibitor wird anhand der Korsmeyer-Peppas-Gleichung berechnet nach der Formel (5)

$$M_t/M_\infty = k \cdot t^n, \qquad (5)$$

wobei $M_t/M_\infty$ die partielle Stofffreisetzung ist, $t$ ist die Freisetzungszeit, k ist eine kinetische Konstante, die charakteristisch für das Wirkstoff-Polymer-System ist, und n ist ein Exponent, der den Mechanismus der Wirkstoff-Freisetzung charakterisiert.

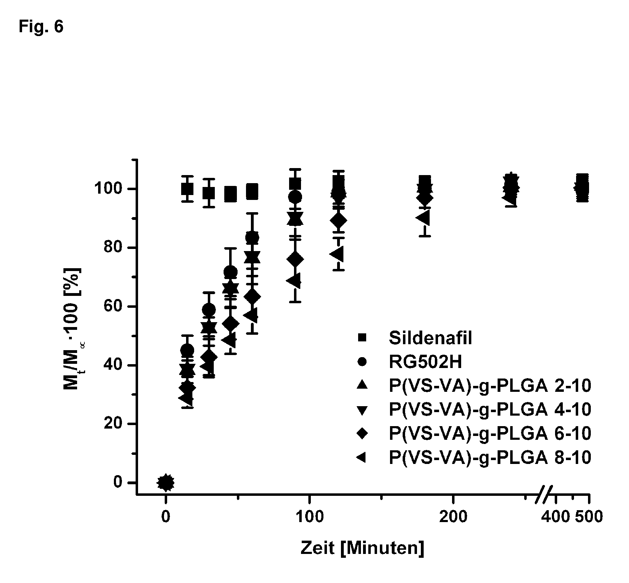

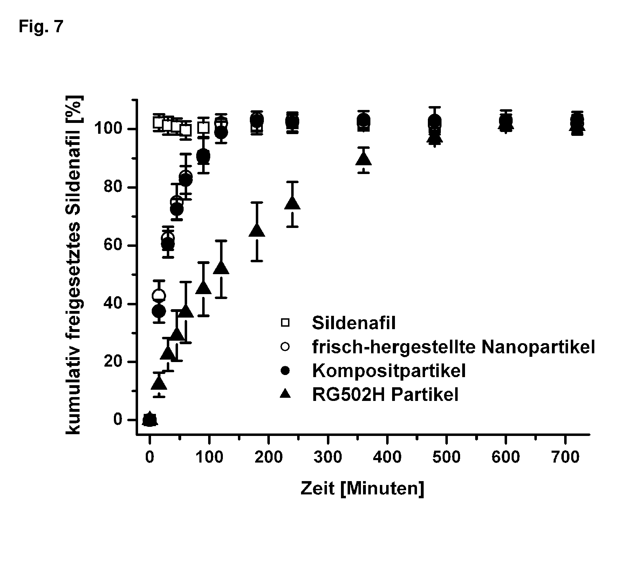

Die Freisetzung des PDE-5-Inhibitors Sildenafil aus den erfindungsgemäßen biokompatiblen Nanopolymerpartikeln *in vitro* erfolgt über einen Zeitraum bis zu 500 Minuten (Fig. 6). Die Freisetzung aus Partikeln mit dem Polymer RG502H erfolgt dabei über bis zu 90 Minuten, die Freisetzung aus Partikeln mit dem Polymer P(VS-VA)-g-PLGA 8-10 erfolgt über einen Zeitraum bis zu 500 Minuten; die Freisetzungszeit aus den weiteren erfindungsgemäßen Partikeln mit den Polymeren P(VS-VA)-g-PLGA 2-10, P(VS-VA)-g-PLGA 4-10 und P(VS-VA)-g-PLGA 6-10 liegt zwischen 90 und 500 Minuten (Fig. 6). In diesem Zeitraum werden >95% Sildenafil aus den erfindungsgemäßen Partikeln freigesetzt. Die Verneblung mit dem Aeroneb® Professional hat dabei keinen Einfluß auf die Freisetzungsrate von Sildenafil. Auch eine sich an den Herstellungsprozess gemäß Ausführungsbeispiel 1.1 anschließende Sprühtrocknung hat keinen Einfluss auf die Freisetzungskinetik von Sildenafil (Abb. 7; schwarze Kreise (mit Sprühtrocknung) im Vergleich zu weißen Kreisen (ohne Sprühtrocknung)).

Alternativ werden die Untersuchungen zur Freisetzung aus den durch Sprühtrockung gemäß Ausführungsbeispiel 1.2 hergestellten erfindungsgemäßen Partikeln mit einem Gehalt von 6% Sildenafil in phosphatgepufferter Salzlösung bei einem pH-Wert von beispielsweise 7,4 unter Zugabe von 0, 1 % Natriumdodecylsulfat (SDS) 700 Minuten lang bei 37°C durchgeführt. Aliquots werden zu den in Fig. 7 angegebenen Zeitpunkten entnommen, zentrifugiert, und die kumulative Freisetzung von Sildenafil wird durch UV/Vis-Spektroskopie wie unter Punkt 2.3 beschrieben bestimmt. Werden die erfindungsgemäßen biokompatiblen Partikel durch Sprühtrocknung gemäß Ausführungsbeispiel 1.2 hergestellt (RG502H Partikel), so erfolgt die Freisetzung des Wirkstoffs Sildenafil über einen Zeitraum bis zu 480 Minuten (Fig. 7; schwarze Dreiecke), während Partikel, die gemäß Ausführungsbeispiel 1.1 hergestellt werden (weiße Kreise) und alternativ anschließend sprühgetrocknet werden (Komposit-Partikel; schwarze Kreise), Sildenafil über einen Zeitraum bis zu 90 Minuten freisetzen (Fig. 7).

**Abbildungslegenden**

Fig. 1
Größenverteilung der erfindungsgemäßen biokompatiblen Nanopolymerpartikel, die durch dynamische Lichtstreuung (DLS) bestimmt wird. Die schwarze Linie repräsentiert die Dichteverteilung der Partikelgrößen, die gestrichelte Linie stellt die kumulative Verteilung der Partikelgrößen dar.

Fig. 2
Stabilität der erfindungsgemäßen biokompatiblen Nanopolymerpartikel bei Vernebelung mit dem Vernebler Aeroneb® Professional. Die Stabilität ist gezeigt als Verhältnis der finalen zu den initialen Eigenschaften der erfindungsgemäßen Partikel (Eigenschaft$_f$/Eigenschaft$_i$) (A) (PDI = Polydispersitätsindex). Teilmengen der Suspensionen von biokompatiblen Nanopolymerpartikeln werden während der Vernebelung für die Analyse der Stabilität bei Vernebelung gesammelt. Die Werte sind als Durchschnittswerte $\pm$ Standardabweichung angegeben (n = 4).

Fig. 3
Adsorbierte Polyvinylalkohol (PVA)-Schichtdicken auf den erfindungsgemäßen biokompatiblen Nanopolymerpartikeln als Funktion der PVA-Konzentration (C$_{PVA}$) (A) und Zeta-Potenzial der biokompatiblen Nanopolymerpartikel, die in PVA-Lösung hergestellt wurden, als Funktion der Elektrolyt-Konzentration (B). Der Anstieg ($_K$) von ln|Zeta-Potenzial| gegen $3.33 \cdot c^{1/2}$ (Konzentration) ergibt die Dicke der adsorbierten Polymerschichten (C). Weiße und schwarze Quadrate in (B) und (C) repräsentieren die Charakteristika der frisch präparierten (B) bzw. vernebelten (C) biokompatiblen Nanopolymerpartikel. Die gerade Linie in (C) repräsentiert den linearen Verlauf der experimentellen Daten ($R^2$ >0,99). Die adsorbierte PVA-Schicht ist in der repräsentativen transmissionselektronenmikroskopischen Aufnahme (D) sichtbar (Maßstab = 20 nm). Die Werte sind als Durchschnittswerte $\pm$ Standardabweichung angegeben (n = 4).

Fig. 4
Sildenafil-Gehalt der erfindungsgemäßen biokompatiblen Nanopolymerpartikel, die mit 1% PVA gemäß Ausführungsbeispiel 1 bei verschiedenen pH-Werten (Kreise = pH 4; Quadrate = pH 8) hergestellt werden. Der Sildenafil-Gehalt ist in Abhängigkeit vom theoretischen Sildenafil-Gehalt dargestellt. Die Werte sind als Durchschnittswerte $\pm$ Standardabweichung angegeben (n = 4).

Fig. 5
Sildenafil-Gehalt der erfindungsgemäßen biokompatiblen Nanopolymerpartikel, die mit verschiedenen linearen PLGA-Copolymeren (RG502H, RG502, RG503H oder RG504H) (A) oder verzweigten (P(VS-VA)-g-PLGA)-Copolymeren (B) gemäß Ausführungsbeispiel 1 mit einem theoretischen Sildenafil-Gehalt von 10% hergestellt werden. Die Werte sind als Durchschnittswerte $\pm$ Standardabweichung angegeben (n = 4). Die Sternchen über den Balken zeigen statistisch relevante Differenzen im Vergleich mit dem PLGA-Copolymer RG502H an (p<0,05).

Fig. 6
Profile der Freisetzung *in vitro* von Sildenafil aus den erfindungsgemäßen biokompatiblen Nanopolymerpartikeln.

Teilmengen von Suspensionen der biokompatiblen Nanopolymerpartikel werden während der Vernebelung für die Analyse des Einflusses der Verneblung auf das Freisetzungsprofil von Sildenafil aus den biokompatiblen Nanopolymerpartikeln gesammelt. Die jeweils verwendeten PLGA- oder P(VS-VA)-g-PLGA-Copolymere werden durch unterschiedliche Symbole entsprechend der Legende dargestellt. Als Vergleich ist das Lösungsprofil von freiem Sildenafil (schwarze Quadrate) mit angegeben. Die Werte sind als Durchschnittswerte ± Standardabweichung angegeben (n = 4).

Fig. 7
Kumulative *in vitro*-Freisetzung des Wirkstoffs Sildenafil aus Partikeln, die durch Sprühtrocknung hergestellt werden (RG502H Partikel; schwarze Dreiecke) im Vergleich zu frisch präparierten Nanopartikeln, welche gemäß Ausführungsbeispiel 1.1 hergestellt werden (weiße Kreise), sowie im Vergleich zu frisch präparierten Nanopartikeln, welche gemäß Ausführungsbeispiel 1.1 hergestellt und anschließend aus wässriger Lösung sprühgetrocknet werden (Komposit-Partikel; schwarze Kreise) und im Vergleich zu freiem Sildenafil (weiße Quadrate). Die Werte sind als Durchschnittswerte ± Standardabweichung angegeben (n = 3).

**Patentansprüche**

1. Biokompatible Nanopolymerpartikel zur Herstellung eines Mittels für die Behandlung der pulmonalen arteriellen Hypertonie oder der erektilen Dysfunktion, **dadurch gekennzeichnet, dass** sie aus einem biokompatiblen Polymer und einem Stabilisator und einem Wirkstoff zur Behandlung der pulmonalen Hypertonie oder erektilen Dysfunktion bestehen.

2. Biokompatible Nanopolymerpartikel gemäß Anspruch 1, **dadurch gekennzeichnet, dass** das biokompatible Polymer ausgewählt ist aus einem Polyester, Polyanhydrid, Polyorthoester, Polyphosphorester, Polycarbonat, Polyketal, Polyharnstoff, Polyurethan, Blockcopolymer (PEG-PLGA), Sternpolymer oder Kammpolymer.

3. Biokompatible Nanopolymerpartikel gemäß Anspruch 2, **dadurch gekennzeichnet, dass** das Polyester Poly(D,L-laktid-*co*-glykolid)-Copolymer (PLGA) ist.

4. Biokompatible Nanopolymerpartikel gemäß Anspruch 2, **dadurch gekennzeichnet, dass** das Kammpolymer Poly (vinylsulfonat-co-vinylalkohol)-*graft*-poly(D,L-laktid-*co*-glykolid)-Copolymer (P(VS-VA)-g-PLGA) oder Sulfobutyl-polyvinylalkohol-*graft*-poly(laktid-*co*-glykolid)-Copolymer (SB-PVA-g-PLGA) ist.

5. Biokompatible Nanopolymerpartikel gemäß Anspruch 1, **dadurch gekennzeichnet, dass** der Stabilisator ausgewählt ist aus der Gruppe der nichtionischen Tenside, anionischen Tenside, amphoteren Tenside oder Polymere.

6. Biokompatible Nanopolymerpartikel gemäß Anspruch 5, **dadurch gekennzeichnet, dass** das Polymer Polyvinylalkohol (PVA) ist.

7. Biokompatible Nanopolymerpartikel gemäß Anspruch 1, **dadurch gekennzeichnet, dass** der Wirkstoff ausgewählt ist aus der Gruppe der Phosphodiesterase-Inhibitoren (PDE-Inhibitoren) oder der Guanylat-Cyclase-Aktivatoren oder Guanylat-Cyclase-Stimulatoren oder der Endothelin-Rezeptor-Antagonisten oder der Prostanoide.

8. Biokompatible Nanopolymerpartikel gemäß Anspruch 7, **dadurch gekennzeichnet, dass** der PDE-Inhibitor der PDE-5-Inhibitor Sildenafil ist.

9. Biokompatible Nanopolymerpartikel gemäß Anspruch 8, **dadurch gekennzeichnet, dass** das Sildenafil als freie Base vorliegt.

10. Biokompatible Nanopolymerpartikel gemäß einem der Ansprüche 1 bis 9, **dadurch gekennzeichnet, dass** sie mit piezoelektrischen, Düsen-, oder Ultraschall-Aerosolgeneratoren, Soft-Mist-Inhalern, Metered Dose Inhalern oder Dry Powder Inhalern vernebelbar sind.

11. Biokompatible Nanopolymerpartikel gemäß einem der Ansprüche 1 bis 10, **dadurch gekennzeichnet, dass** sie

   - einen Durchmesser zwischen 10 nm bis 10 μm aufweisen und
   - eine Stabilisator-Schichtdicke zwischen 0 bis 50 nm aufweisen und

- zwischen 0 bis 50 % (w/w) eines Wirkstoffs zur Behandlung der pulmonalen Hypertonie oder der erektilen Dysfunktion enthalten und
- den Wirkstoff über einen Zeitraum von bis zu 48 Stunden freisetzen.

**12.** Biokompatible Nanopolymerpartikel gemäß einem der Ansprüche 1 bis 11, **dadurch gekennzeichnet, dass** sie besonders bevorzugt einen Durchmesser zwischen 500 nm bis 5 $\mu$m für eine längerdauernde Wirkstoff-Freisetzung oder zwischen 50 nm und 250 nm zur Verhinderung der Aufnahme der Partikel durch Makrophagen aufweisen.

**13.** Biokompatible Nanopolymerpartikel gemäß einem der Ansprüche 1 bis 12, **dadurch gekennzeichnet, dass** sie besonders bevorzugt zwischen 1 bis 20 % (w/w) eines Wirkstoffs zur Behandlung der pulmonalen Hypertonie oder der erektilen Dysfunktion aufweisen.

**14.** Verfahren zur Herstellung von biokompatiblen Nanopolymerpartikeln gemäß einem der Ansprüche 1 bis 13, **gekennzeichnet durch** die Schritte

    a) Lösen des biokompatiblen Polymers und des Wirkstoffs in einem Lösungsmittel unter Bildung einer organischen Phase,
    b) Emulgieren der organischen Phase in einer wässrigen Phase enthaltend einen Stabilisator,
    c) Vermischen der organischen und der wässrigen Phase,
    d) Entfernen des Lösungsmittels und Erhalten der Partikel in Suspension.

**15.** Verfahren zur Herstellung von biokompatiblen Nanopolymerpartikeln gemäß einem der Ansprüche 1 bis 13, **gekennzeichnet durch** die Schritte

    a) Lösen des biokompatiblen Polymers und des Wirkstoffs in einem Lösungsmittel unter Bildung einer organischen Phase,
    b) Sprühtrocknung der organischen Phase.

**16.** Verfahren zur Herstellung von biokompatiblen Nanopolymerpartikeln gemäß einem der Ansprüche 14 bis 15, **dadurch gekennzeichnet, dass** das biokompatible Polymer ein Polyester, Polyanhydrid, Polyorthoester, Polyphosphorester, Polycarbonat, Polyketal, Polyharnstoff, Polyurethan, Blockcopolymer (PEG-PLGA), Sternpolymer oder Kammpolymer ist.

**17.** Verfahren zur Herstellung von biokompatiblen Nanopolymerpartikeln gemäß einem der Ansprüche 14 bis 15, **dadurch gekennzeichnet, dass** der Stabilisator ein nichtionisches Tensid, anionisches Tensid, amphoteres Tensid oder Polymer ist.

**18.** Verfahren zur Herstellung von biokompatiblen Nanopolymerpartikeln gemäß einem der Ansprüche 14 bis 15, **dadurch gekennzeichnet, dass** der Wirkstoff ein Phosphodiesterase-Inhibitor (PDE-Inhibitor) oder Guanylat-Cyclase-Aktivator oder Guanylat-Cyclase-Stimulator oder Endothelin-Rezeptor-Antagonist oder Prostanoid ist.

**19.** Verwendung von biokompatiblen Nanopolymerpartikeln gemäß einem der Ansprüche 1 bis 18 zur Herstellung eines pharmazeutischen Mittels für die Behandlung von pulmonaler Hypertonie.

**20.** Verwendung von biokompatiblen Nanopolymerpartikeln gemäß einem der Ansprüche 1 bis 18 zur Herstellung eines pharmazeutischen Mittels für die Behandlung von erektiler Dysfunktion.

**21.** Verwendung von biokompatiblen Nanopolymerpartikeln gemäß Anspruch 19, **dadurch gekennzeichnet, dass** das pharmazeutische Mittel durch Inhalation, Instillation, ein Bronchoskop oder einen Beatmungszugang verabreicht wird.

**Fig. 1**

**Fig. 2**

Fig. 3 (A)

**Fig. 3 (B)**

**Fig. 3 (C), (D)**

**Fig. 4**

**Fig. 5 (A)**

**Fig. 5 (B)**

**Fig. 6**

**Fig. 7**

Europäisches
Patentamt

European
Patent Office

Office européen
des brevets

**EUROPÄISCHER RECHERCHENBERICHT**

Nummer der Anmeldung

EP 11 16 7486

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 140 882 A1 (UNIV KYUSHU NAT UNIV CORP [JP]; KOWA CO [JP]) 6. Januar 2010 (2010-01-06) | 1-3,5-7, 10, 12-14, 16-19,21 | INV. A61K9/00 A61K31/519 |
| Y | * Absatz [0016] - Absatz [0026] * * Beispiel 1 * ----- | 1-21 | |
| X | WO 2004/046202 A2 (TRANSMIT TECHNOLOGIETRANSFER [DE]; UNIV GIESSEN JUSTUS LIEBIG [DE]; UN) 3. Juni 2004 (2004-06-03) | 1-5,7, 10,19,21 | |
| Y | * Beispiele 1-3 * * Ansprüche 1-23 * ----- | 1-21 | |
| X | WO 2010/009146 A1 (UNIV KANSAS [US]; BERKLAND CORY J [US]; BAILEY MARK [US]; EL GENDY NAS) 21. Januar 2010 (2010-01-21) * Beispiele 1-9 * * Seite 18, Zeile 26 * ----- | 1-21 | |

RECHERCHIERTE
SACHGEBIETE (IPC)

A61K

Der vorliegende Recherchenbericht wurde für alle Patentansprüche erstellt

| Recherchenort | Abschlußdatum der Recherche | Prüfer |
|---|---|---|
| München | 25. Januar 2012 | Sindel, Ulrike |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
...........................................................................
& : Mitglied der gleichen Patentfamilie, übereinstimmendes
   Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 11 16 7486

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

25-01-2012

| Im Recherchenbericht angeführtes Patentdokument | Datum der Veröffentlichung | Mitglied(er) der Patentfamilie | Datum der Veröffentlichung |
|---|---|---|---|
| EP 2140882 A1 | 06-01-2010 | CA 2685054 A1<br>EP 2140882 A1<br>KR 20100015758 A<br>US 2010086615 A1<br>WO 2008139703 A1 | 20-11-2008<br>06-01-2010<br>12-02-2010<br>08-04-2010<br>20-11-2008 |
| WO 2004046202 A2 | 03-06-2004 | AU 2003294626 A1<br>CA 2545877 A1<br>DE 10253623 A1<br>EP 1562559 A2<br>US 2006127485 A1<br>WO 2004046202 A2 | 15-06-2004<br>03-06-2004<br>03-06-2004<br>17-08-2005<br>15-06-2006<br>03-06-2004 |
| WO 2010009146 A1 | 21-01-2010 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **DAILEY LA ; KLEEMANN E ; WITTMAR M et al.** Surfactantfree, biodegradable nanoparticles for aerosol therapy based on the branched polyesters, DEAPA-PVAL-g-PLGA. *Pharm. Res.,* 2003, vol. 20 (12), 2011-2020

- **DAILEY LA ; SCHMEHL T ; GESSLER T et al.** Nebulization of biodegradable nanoparticles: impact of nebulizer technology and nanoparticle characteristics on aerosol features. *J. Controlled Release.,* 2003, vol. 86 (1), 131-144